# EUROPÄISCHE PATENTSCHRIFT

(11) **EP 1 839 645 B1**
(45) Veröffentlichungstag und Bekanntmachung des Hinweises auf die Patenterteilung: **03.03.2010**
(21) Anmeldenummer: 07103569.5
(22) Anmeldetag: 06.03.2007
(51) Int. Cl.: A61K 8/97, A61K 8/19, A61K 8/42, A61Q 19/00

(54) **Kosmetische oder dermatologische Emulsionen enthaltend Licochalcon A oder einen Licochalcon A enthaltenden Extrakt aus Radix Glycyrrhizae inflatae**
Cosmetic or dermatological emulsions containing licochalcone A or an extract of radix glycyrrhizae inflatae containing licochalcone A
Emulsions cosmétiques ou dermatologiques comportant du licochalcone A ou un extrait comprenant du licochalcone A à partir de radix glycyrrhizae inflatae

(30) Priorität: 06.03.2006 DE 102006010589
(43) Veröffentlichungstag der Anmeldung: 03.10.2007
(73) Patentinhaber: Beiersdorf AG, 20253 Hamburg (DE)
(72) Erfinder: Kolbe, Ludger, 21255, Dohren (DE); Traupe, Bernd, 24568, Kaltenkirchen (DE); Filbry, Alexander, 22459, Hamburg (DE); Lüdemann, Dorthe, 21039, Escheburg (DE); Behrens, Svea, 21109 Hamburg (DE); Bleckmann, Andreas, 22926 Ahrensburg (DE); Meiring, Uta, 21077 Hamburg (DE)

(56) Entgegenhaltungen:
- EP-A- 1 529 520
- EP-A- 1 537 849
- WO-A-03/101414
- WO-A-2005/020915
- WO-A-2005/027866
- DATABASE IP.COM [Online] IP.com number: IPCOM000134374D 3. März 2006 (2006-03-03), "Cosmetic compositions containing glucanes and further ingredients" XP013113050 gefunden im WWW.IP.COM

## Beschreibung

Gegenstand der vorliegenden Erfindung sind kosmetische oder dermatologische Emulsionen mit einem Gehalt an Licochalcon A bzw. einem Licochalcon A enthaltenden Extrakt aus *Radix Glycyrrhizae inflatae* und Silber Dihydrogen Citrat.

Die Haut, insbesondere die Epidermis, ist als Barriereorgan des menschlichen Organismus in besonderem Maße äußeren Einwirkungen unterworfen. Nach dem heutigen wissenschaftlichen Verständnis repräsentiert die Haut ein immunologisches Organ, das als immunkompetentes peripheres Kompartiment eine eigene Rolle in induktiven, effektiven und regulativen Immunprozessen des Gesamtorganismus spielt.

Die Epidermis ist reich mit Nerven und Nervenendapparaten wie Vater-Pacini-Lamellenkörpern, Merkel-Zell-Neuritenkomplexen und freien Nervenendigungen für Schmerz-, Kälte-, Wärmeempfindung und Juckreiz ausgestattet.

Typische u. a. mit dem Begriff "empfindliche Haut" in Verbindung gebrachte, störende neurosensorische Phänomene sind Hautrötung, Kribbeln, Prickeln, Spannen und Brennen der Haut und Juckreiz. Sie können durch stimulierende Umgebungsbedingungen - z. B. Massage, Einwirkung (waschaktiver) Tenside, Wettereinfluss wie Sonne, Kälte, Trockenheit, aber auch feuchte Wärme, Wärmestrahlung und UV-Strahlung, z. B. der Sonne - hervorgerufen werden.

Erythematöse Hauterscheinungen treten ferner auch als Begleiterscheinungen bei gewissen Hauterkrankungen oder -unregelmäßigkeiten auf. Beispielsweise ist der typische Hautausschlag beim Erscheinungsbild der Akne und dem atopischem Ekzem sowie der Neurodermitis regelmäßig mehr oder weniger stark gerötet.

Auch die Rasur induziert in dafür empfindlichen Personen Erytheme, Brennen, Juckreiz und Spannungsgefühle, die durch die oberflächliche Verletzung und die mechanische Belastung der obersten Hautschichten sowohl bei der Nassrasur als auch bei der Trockenrasur ausgelöst werden. Diese Beschwerden treten häufig bei der täglichen Rasur der Barthaare auf, aber auch nach der Rasur von Achsel-, Scham-, und Beinbehaarung können Irritationen auftreten.

Neben den positiven Auswirkungen des Sonnenlichtes, wie dem allgemeinen Wohlbefinden, der Bildung von Vitamin D3 und der Aknebehandlung, gibt es auch negative Auswirkungen, denen es entgegenzuwirken gilt.

Die Bedingungen eines Sonnenbades stellen für den menschlichen Organismus eine ungewohnte, zum Teil extreme Belastung dar, von der insbesondere die Haut betroffen ist. Solange die Strahlenbelastung ein gewisses Ausmaß nicht überschreitet, wird unsere Haut damit fertig. Geringere Schäden, wie sie bei nicht spürbaren Suberythemen vorliegen, werden sofort behoben.

Zur Behandlung der Haut dienen die so genannten Aftersun-Präparate, deren Anwendung grundsätzlich nach jeder Sonnenexposition empfohlen wird. Verwendet werden hier häufig Emulsionen oder wässrige Hydrogele, die neben üblichen Feuchthaltesubstanzen auch spezielle Wirkstoffe enthalten können, wie beispielsweise
- kühlende Stoffe,
- lokal anaestesierende Stoffe und/oder
- desinfizierende Stoffe, um mögliche Hautinfektionen zu verhindern.

WO-03/101414, WO-2005/027866 und EP-1529520 offenbaren kosmetische Zubereitungen, die Licochalcon A enthalten. WO-2005/020915 offenbart Silberdihydrogencitrat als antimikrobiell wirkende Substanz.

Aufgabe der vorliegenden Erfindung war es, Zubereitungen gegen trockene Haut, juckende trockene Haut oder atopisch trockene Haut zur Verfügung zu stellen, welche insbesondere auch bei empfindlicher und/oder (mechanisch) belasteter Haut angewendet werden können und dabei möglichst die damit verbundenen negativen Erscheinungsbilder lindern bzw. verbessern.

Es war überraschend und darin liegt die Lösung dieser Aufgaben, dass kosmetische oder dermatologische Emulsionen mit einem Gehalt an
a) Licochalcon A und/oder einem Licochalcon A enthaltenden Extrakt aus *Radix Glycyrrhizae inflatae*
   und
b) Silber Dihydrogen Citrat
den Nachteilen des Standes der Technik abhelfen würden.

Die Emusionen gemäß der Erfindung sind in jeglicher Hinsicht überaus befriedigende Präparate, die sich durch eine hervorragende Wirkung auszeichnen. Bei Anwendung der erfindungsgemäßen Emulsionen ist eine wirksame Behandlung, aber auch eine Prophylaxe von entzündlichen Hautzuständen - auch dem atopischen Ekzem - und/oder der Schutz der Haut bei empfindlich determinierter trockener Haut möglich. Die erfindungsgemäßen Emulsionen können aber in überraschender Weise auch zur Beruhigung von empfindlicher oder gereizter Haut dienen.

Es war für den Fachmann nicht vorauszusehen gewesen, dass die erfindungsgemäßen Emusionen
- lichtstrapazierte Haut oder
- von der Rasur strapazierte Haut besser pflegen,
- die Nachreaktionen der Haut auf die Einwirkung von UV-Strahlung besser vermindern,
- die vom Sonnenbaden gereizte Haut besser beruhigen,
- leichten Sonnenbrand schneller zum Abklingen bringen würden,
- besser als feuchtigkeitsspendende Zubereitungen wirken,
- auch die gereizte Haut von Neurodermitikern wirksam beruhigen würden,
- besser die Hautglättung fördern und
- sich durch besser Pflegewirkung auszeichen würden
als die Zubereitungen des Standes der Technik.

Die Erfindung ist selbstverständlich nicht auf Emusionen beschränkt, welche nach dem Sonnenbad angewendet werden, sondern umfasst naturgemäß alle kosmetischen und dermatologischen Anwendungen, bei welchen eine hautberuhigende oder entzündungslindernde Wirkung gewünscht oder von Vorteil sein könnte.

Hier sei neben der Neurodermitis insbesondere auch der Rasurbrand genannt, wie er nach der Rasur häufig auftritt.

Es ist vorteilhaft im Sinne der vorliegenden Erfindung, wenn die Emulsionen 0,0001 bis 5 Gew.-%, insbesondere 0,001 bis 1 Gew.-%, ganz besonders 0,005 bis 0,15 Gew.-% Licochalcon A enthalten, bezogen auf das Gesamtgewicht der Emulsion.

Es ist erfindungsgemäß ferner vorteilhaft, wenn die Emusionen Licochalcon A als Bestandteil von pflanzlichen Extrakten, insbesondere von *Radix Glycyrrhizae inflatae,* enthalten.

Die Pflanzenart *Glycyrrhiza inflata* gehört wie das in Europa offizinelle Süßholz *Glycyrrhiza glabra* der Gattung *Glycyrrhiza* an, die zur Pflanzenfamilie der Fabaceae (Erbsengewächse) gehört. Die Droge *Radix Glycyrrhizae inflatae,* d. h., die Wurzel der Pflanze, ist, beispielsweise in der fernöstlichen Medizin, gebräuchlich. Die Verwendung der Droge als Entzündungshemmer ist ebenfalls bekannt.

Ein Bestandteil des wässrigen Auszugs aus Radix Glycyrrhizae inflatae ist das Licochalcon A, welches sich durch die folgende Strukturformel auszeichnet:

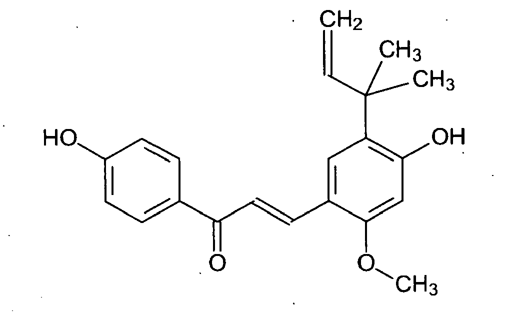

Es wird angenommen, dass diese Substanz, möglicherweise in Synergie mit den übrigen Bestandteilen des Extraktes, Anteil an der erfindungsgemäßen Wirkung besitzt.

In dem Fall, dass die erfindungsgemäßen Emulsionen einen wässrigen Extrakt aus *Radix Glycyrrhizae inflatae* enthalten, ist es vorteilhaft, die Menge an diesem Extrakt aus dem Bereich von 0,001 bis 10 Gew.-%, insbesondere 0,05 bis 5 Gew.-%, ganz besonders 0,01 bis 2 Gew.-% zu wählen -jeweils bezogen auf das Gesamtgewicht der Emulsion.

Ganz besonders vorteilhaft ist es, von einem wässrigen Extrakt auszugehen, der unter der Bezeichnung Aqua Licorice Extract P-U der Firma *Maruzen* auszugehen, der ein wässriges Gemisch (ca. 10 Gew.-% Wasser) aus *Radix Glycyrrhizae inflatae* (ca. Gew.-%, Anteil Licochalcone A im Extrakt ca. 22 %, PPG-6 Decyltetradeceth-30 (ca. 25 Gew.-%) und Butylenglycol (ca. 60 Gew.-%) darstellt.

Ferner ist es vorteilhaft, Licochalcone A in anderen Vehikelsystemen in einer Konzentration von 0,0001 bis 5 Gew.-%, insbesondere 0,001 bis 1 Gew.-%, ganz besonders 0,005 - 0,05 Gew.-% zu verwenden.

Silber Dihydrogen Citrat hat die chemische Summenformel AgC₆H₇O₇ und zeichnet sich durch die folgende Strukturformel aus:

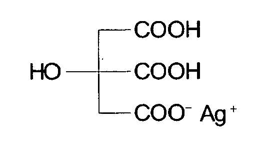

Es ist vorteilhaft im Sinne der vorliegenden Erfindung, wenn die Emulsionen 0,05 bis 0,5 Gew.-%, insbesondere 0,1 bis 0,3 Gew.-% Silber Dihydrogen Citrat enthalten, bezogen auf das Gesamtgewicht der Emulsion.

Besonders vorteilhaft im Sinne der vorliegenden Erfindung ist Silber Dihydrogen Citrat, welches unter der Handelsbezeichnung TINOSAN^{®} SDC bei der Fa. Ciba Specialty Chemicals erhältlich ist.

In einer besonders vorteilhaften Ausführungsform können die erfindungsgemäßen Emulsionen ein oder mehrere Derivate ungesättigter Fettsäuren enthalten, wobei das Fettsäurederivat mindestens eine ungesättigte Fettsäure enthält. Es kann aber auch mehrere ungesättigte Fettsäuren enthalten. So können Triglyceride z. B. eine, zwei oder drei ungesättigte Fettsäuren enthalten.

Erfindungsgemäß bevorzugt sind Fettsäurederivate einfach oder mehrfach ungesättigter Carbonsäuren mit vorzugsweise 16 bis 24 Kohlenstoffatomen und insbesondere 1 bis 6 Doppelbindungen, besonders bevorzugt mit einer Doppelbindung oder zwei oder drei Doppelbindungen. Die ungesättigten Fettsäuren können sowohl der n-6-Serie als auch der n-3-Serie angehören. Bevorzugt werden Fettsäuren der n-6-Serie. Besonders bevorzugt sind Derivate, die ungesättigte Carbonsäuren der Kettenlänge C16 bis C18 mit einer Doppelbindung, Carbonsäuren der Kettenlänge C16 bis C20 mit 2 Doppelbindungen, Carbonsäuren der Kettenlänge C18 bis C20 mit 3 Doppelbindungen und Carbonsäuren der Kettenlänge C20 bis C22 mit 5 oder 6 Doppelbindungen enthalten. Die Doppelbindungen können sowohl isoliert als auch konjugiert vorliegen. Besonders bevorzugte Fettsäuren mit einer Doppelbindung sind Ölsäure und Palmitoleinsäure. Bei den Fettsäuren mit 2 Doppelbindungen ist besonders bevorzugt Linolsäure, bei denen mit 3 Doppelbindungen die alpha- und gamma-Linolensäure, die Dihomogammalinolensäure und Arachidonsäure und bei denen mit 5 oder 6 Doppelbindungen die Eicosapentaensäure und Docosahexänsäure.

Es ist vorteilhaft im Sinne der vorliegenden Erfindung, wenn die Fettsäurederivate Ester darstellen, wie z. B. Glyceride sowie insbesondere Triglyceride oder Ester mit Alkoholen, insbesondere Monoalkanole mit vorzugsweise 1 bis 5 Kohlenstoffatomen, besonders bevorzugt Ethylester, insbesondere Triglyceride und Ethylester der namentlich genannten Fettsäuren. Die Triglyceride können sowohl 3 gleiche Fettsäuren als auch eine oder 2 oder 3 verschiedenartige Fettsäuren enthalten.

Besonders bevorzugte Verbindungen sind die Ethylester und Triglyceride der Linolsäure, der alpha- und gamma-Linolensäure, der Dihomogammalinolensäure sowie der Eicosapentaensäure und Docosahexaensäure, insbesondere aber der Gammalinolensäure.

Die Fettsäurederivate können sowohl synthetisch hergestellt als auch in natürlich vorkommender Form - z. B. als Pflanzenöle - eingesetzt werden. Erfindungsgemäß bevorzugte Öle, die ungesättigte Fettsäurederivate als Triglyceride enthalten, sind Nachtkerzensamenöl (Oenothera Biennis), Borretschöl, Traubenkernöl, Erdnussöl, Weizenkeimöl, Schwarzes Johannisbeersamenöl und Fischöle.

Der Anteil der Fettsäurederivate in den erfindungsgemäßen Emulsionen kann z. B. zwischen 0,1 bis 20 Gew.-%, vorzugsweise 0,5 bis 15 Gew.-%, insbesondere aber 1 bis 10 Gew.-% betragen, jeweils bezogen auf das Gesamtgewicht der Emulsion.

Die Ölphase der erfindungsgemäßen Emusionen kann ferner folgende Bestandteile enthalten.

Diese werden vorteilhaft gewählt aus der Gruppe der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 bis 18 C-Atomen. Besonders vorteilhaft im Sinne der vorliegenden Erfindung ist Capryl-Caprinsäuretriglycerid (INCI: caprylic/capric triglyceride) zu verwenden, insbesondere wenn die Zubereitung im Sinne der vorliegenden Erfindung auch Cyclomethicon enthält.

Die Fettsäuretriglyceride können vorteilhaft gewählt werden aus der Gruppe der synthetischen, halbsynthetischen und natürlichen Öle, wie z. B. Olivenöl, Sonnenblumenöl, Sojaöl, Erdnussöl, Rapsöl, Mandelöl, Palmöl, Kokosöl, Rizinusöl, Weizenkeimöl, Traubenkernöl, Distelöl, Nachtkerzenöl und dergleichen mehr.

Die Ölphase kann im Sinne der vorliegenden Erfindung ferner vorteilhaft Substanzen aus der Gruppe der Ester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen sowie aus der Gruppe der Ester aus aromatischen Carbonsäuren und gesättigten und/oder ungesättigten, verzweigten und/oder unverzweigten Alkoholen einer Kettenlänge von 3 bis 30 C-Atomen enthalten. Solche Esteröle können dann vorteilhaft gewählt werden aus der Gruppe Isopropylpalmitat, Isopropylstearat, n-Butylstearat, n-Hexyllaurat, Isooctylstearat, Isononylstearat, Isononylisononanoat, 2-Ethylhexylpalmitat, 2-Hexyldecylstearat, 2-Octyldodecylpalmitat, Erucylerucat sowie synthetische, halbsynthetische und natürliche Gemische solcher Ester, wie z. B. Jojobaöl.

Die Bestandteile der Ölphase können beliebige Abmischungen von Öl-, Fett- und/oder Wachskomponenten darstellen.

Es ist vorteilhaft im Sinne der vorliegenden Erfindung, wenn die Emusionen einen oder mehrere Glycerinester, insbesondere Glycerinester von α-Hydroxycarbonsäuren und gesättigten Fettsäuren enthalten, wobei die Gesamtmenge der Glycerinester in den fertigen kosmetischen Emusionen vorteilhaft aus dem Bereich von 0,1 bis 10,0 Gew.-%, bevorzugt 0,5 bis 6,0 Gew.-% gewählt wird, jeweils bezogen auf das Gesamtgewicht der Emusionen.

Ferner ist es vorteilhaft im Sinne der vorliegenden Erfindung, wenn die Emusionen einen oder mehrere Fettalkohole enthalten. Bevorzugter Fettalkohol ist der Cetylalkohol.

Die Gesamtmenge an einem oder mehreren Fettalkoholen in den fertigen kosmetischen Emusionen wird vorteilhaft aus dem Bereich von 0,1 bis 10,0 Gew.-%, bevorzugt 0,5 bis 6,0 Gew.-% gewählt, jeweils bezogen auf das Gesamtgewicht der Emusionen.

Die erfindungsgemäße kosmetische Zubereitung kann erfindungsgemäß vorteilhaft in Form einer Wasser-in-Öl-Emulsion (W/O-Emulsion) vorliegen. In diesem Fall sind die folgende Emulgatoren erfindungsgemäß bevorzugt:

| **Handelsname** | **INCI-Name** |
|---|---|
| Lameform TGI | Polyglyceryl-3 Diisostearate |
| Isolan GI 34 | Polyglyceryl-4 Isostearate |
| Dehymuls PGPH | Polyglyceryl-2 Dipolyhydroxystearate |
| Arlacel P 135 | PEG-30 Dipolyhydroxystearate |
| Eucerit PA | Lanolin Alcohol |
| Atlas G-1049 | PEG-40 Sorbitan Perisostearate |
| Abil EM 90 | Cetyl Dimethicone Copolyol |
| Arlacel 989 | PEG-7 Hydrogenated Castor Oil |
| Elfacos ST 9 | PEG 45/Dodeceyl Glycol Copolymer |
| Elfacos ST 37 | PEG 22/Dodeceyl Glycol Copolymer |
| Isostearinsäure PP | Pentaerythrityl Isostearate |
| Imwitor 780 K | Isostearyl Diglyceryl Succinate |
| Arlacel 987 | Sorbitan Isostearate |
| Hostacerin DGI | Polyglyceryl-2 Sesquiisostearate |
| Tegin ISO | Glyceryl Isostearate |
| Arlacel 60 | Sorbitan Stearate |
| Tegin M | Glyceryl Stearate |
| Arlantone G | PEG-25 Hydrogenated Castor Oil |
| Arlantone T | PEG-40 Sorbitan Peroleate |
| Arlacel 80 | Sorbitan Oleate Cera Microcristallina + Paraffinum Liquidum + Ozokerite+ Hydrogenated Castor Oil + Glyceryl Isostearate + Polyglyceryl-3 Oleate |
| Atlas G-1 049 | PEG-40 Sorbitan Perisostearate |
| ABIL WS 08 | Cetyl Dimethicone Copolyol + Hexyl Laurate + Polyglyceryl-3 Oleate + Cetyl Dimethicone |
| Hostacerin DGO | Polyglyceryl-2 Sesquioleate |
| Abil WE 09 | Cetyl Dimethicone Copolyol (+) Polyglyceryl-4 Isostearate (+) Hexyl Laurate |
| Abil EM 90 | Cetyl PEG/ PPG- 10/1- Dimethicone |
| Abil EM 97 | Dimethicone Copolyol (+) Cyclomethicone |
| Isolan GO 33 | Polyglyceryl-3 Oleate |
| Montanov WO 18 | Isostearyl Alcohol (+) Isostearyl Glucoside |
| Cremophor GO 32 | Polyglyceryl-3 Dioleate |
| Olivem 900 | Sorbitan Oleate |
| Sisterna SP01-C | Sucrose Distearate |
| Sisterna SP10-C | Sucrose Distearate |
| Dehymuls B | Polyglyceryl-3 Diisostearate (+) Glyceryl Oleate |
| Emulsogen SRH | Rapeseed Sorbitols |
| Emulsogen SRO | Rapeseed Sorbitols |
| Rylo PG 11 | Polyglyceryl Dimer Soyate |
| Grindstedt PS 401 (Dermofeel PR) | Polyglyceryl Polyricinoleate |
| Isolan PDI | Polyglyceryl-3 Dimerate |
| Arlacel 986 | Glyceryl Sorbitan Stearate |
| Decaglyn 5-HS | Polyglyceryl-10 Hydroxystearate |

Ganz besonders bevorzugt ist es, wenn der oder die W/O-Emulgatoren gewählt werden aus der Gruppe PEG-30 Dipolyhydroxystearat, Polyglyceryl-3-Diisostearat, PEG-40 Sorbitan Perisostearate.

In einer zweiten erfindungsgemäß vorteilhaften Ausführungsform der vorliegenden Erfindung kann die erfindungsgemäße kosmetische Zubereitung in Form einer Öl-in-Wasser-Emulsion (O/W-Emulsion) vorliegen.

In diesem Fall werden der oder die O/W-Emulgatoren erfindungsgemäß vorzugsweise aus der folgenden Gruppe gewählt:
Glycerylstearat in Kombination mit Ceteareth-20 und/oder Ceteareth-25, Ceteareth-6 in Kombination mit Stearylalkohol, Cetylstearylalkohol in Kombination mit PEG-40-Ricinusöl und Natriumcetylstearylsulfat, Triceteareth-4 Phosphat, Glycerylstearat, Natriumcetylstearylsulfat, Lecithin Trilaureth-4 Phosphat, Laureth-4 Phosphat, Stearinsäure, Propylenglycolstearat SE, PEG-25-hydriertes Ricinusöl, PEG-54-hydriertes Ricinusöl und/oder PEG-6 Caprylsäure/Caprinsäureglyceride, Glyceryloleat in Kombination mit Propylenglycol, PEG-9-Stearat, PEG-20 Stearat, PEG-30-Stearat, PEG-40-Stearat, PEG-100-Stearat, Ceteth-2, Ceteth-20, Polysorbate-20, Polysorbate-60, Polysorbate-65 und/oder Polysorbate-100, Glycerylstearat in Kombination mit PEG-100 Stearat, Glycerylmyristat, Glyceryllaurat, PEG-40-Sorbitanperoleat, Laureth-4, Ceteareth-3 und/oder Isostearylglycerylether, Cetylstearylalkohol in Kombination mit Natrium Cetylstearylsulfat, Laureth-23 und/oder Steareth-2, Glycerylstearat in Kombination mit PEG-30 Stearat, PEG-40-Stearat, Glycol Distearat, PEG-22-Dodecyl Glycol Copolymer, Polyglyceryl-2-PEG-4-Stearat, Ceteareth-12, Ceteareth-20, Ceteareth-30, Methyl-glucosesesquistearat, Steareth-10 und/oder PEG-20-Stearat, Steareth-2 in Kombination mit PEG-8 Distearat, Steareth-21, Steareth-20, Isosteareth-20, PEG-45/ Dodecylglycol-Copolymer, Methoxy-PEG-22/Dodecylglycol-Copolymer, PEG-40-Sorbitanperoleat, PEG-40-Sorbitanperisostearat, PEG-20-Glycerylstearat, PEG-20-Glycerylstearat, PEG-8-Bienenwachs, Polyglyceryl-2-laurat, Isostearyldiglycerylsuccinat, Stearamidopropyl-PG-dimoniumchloridphosphat, Glycerylstearat SE, Ceteth-20, Triethylcitrat, PEG-20-Methylglucosesesquistearat, Glycerylstearatcitrat, Cetylphosphat, Cetearyl Sulfat, Sorbitansesquioleat, Triceteareth-4-Phosphat, Trilaureth-4-Phosphat, Polyglyceryl-methylglucosedistearat, Kaliumcetylphosphat, Polyglyceryl-3 Methylglucose Distearate Isosteareth-10, Polyglyceryl-2-sesquiisostearat, Ceteth-10, Oleth-20 und/oder Isoceteth-20, Glycerylstearat in Kombination mit Ceteareth-20, Ceteareth-12, Cetylstearylalkohol und/oder Cetylpalmitat, Cetylstearylalkohol in Kombination mit PEG-20 Stearat, PEG-30-Stearat, PEG-40-Stearat und/oder PEG-100-Stearat.

Als erfindungsgemäß besonders vorteilhafte O/W-Emulgatoren werden Glycerylstearat, PEG-40 Stearat, Triglycerinmethylglucosedistearat, Polyglyceryl-3 Methylglucose Distearate, Polyethylenglycol(21)stearylether, Polyethylenglycol(2)stearylether, Cetearylglucosid, Glycerylstearatcitrat, Natriumcetearyl-sulfat, Cetearylalkohol und/oder Sorbitanstearat eingesetzt.

Die wässrige Phase der erfindungsgemäßen Emusionen enthält gegebenenfalls vorteilhaft Diole oder Polyole niedriger C-Zahl, sowie deren Ether, vorzugsweise Ethanol, Isopropanol, 1,2-Propandiol, Propylenglykol, Glycerin, Ethylenglykol, Ethylenglykolmonoethyl- oder -monobutylether, Propylenglykolmonomethyl, -monoethyl- oder -monobutylether, Diethylenglykolmonomethyl- oder -monoethylether und analoge Produkte.

Ferner können die Emusionen im Sinne der vorliegenden Erfindung ein oder mehrere Verdickungsmittel enthalten, sofern die im Hauptanspruch geforderten Bedingungen eingehalten werden. Das oder die Verdickungsmittel können vorteilhaft gewählt werden aus der Gruppe Siliciumdioxid, Aluminiumsilikate, Polysaccharide bzw. deren Derivate, z. B. Hyaluronsäure, Xanthangummi, Hydroxypropylmethylcellulose, besonders vorteilhaft aus der Gruppe der Polyacrylate, bevorzugt ein Polyacrylat aus der Gruppe der sogenannten Carbopole, beispielsweise Carbopole der Typen 980, 981, 1382, 2984, 5984, oder auch der Typen ETD (Easy-to-disperse) 2001, 2020, 2050, jeweils einzeln oder in beliebigen Kombinationen untereinander. Es ist aber auch vorteilhaft im Sinne der vorliegenden Erfindung, wenn die Emusionen frei von Verdickungsmitteln sind.

Besonders vorteilhafte Emusionen werden ferner erhalten, wenn als Zusatz- oder Wirkstoffe Antioxidantien eingesetzt werden. Erfindungsgemäß enthalten die Emusionen vorteilhaft eines oder mehrere Antioxidantien. Als günstige, aber dennoch fakultativ zu verwendende Antioxidantien können alle für kosmetische und/oder dermatologische Anwendungen geeigneten oder gebräuchlichen Antioxidantien verwendet werden.

Die Menge der Antioxidantien (eine oder mehrere Verbindungen) in den Emusionen beträgt vorzugsweise 0,001 bis 30 Gew.-%, besonders bevorzugt 0,1 bis 20 Gew.-%, insbesondere 0,5 bis 5 Gew.-%, jeweils bezogen auf das Gesamtgewicht der Zubereitung.

Besonders vorteilhaft im Sinne der vorliegenden Erfindung können öllösliche Antioxidantien eingesetzt werden. Bevorzugte Antioxidantien sind Vitamin E und dessen Derivate sowie Vitamin A und dessen Derivate.

Sofern Vitamin E und/oder dessen Derivate das oder die Antioxidantien darstellen, ist vorteilhaft, deren jeweilige Konzentrationen aus dem Bereich von 0,001 bis 10 Gew.-%, bezogen auf das Gesamtgewicht der Formulierung, zu wählen.

Sofern Vitamin A, bzw. Vitamin-A-Derivate, bzw. Carotine bzw. deren Derivate das oder die Antioxidantien darstellen, ist vorteilhaft, deren jeweilige Konzentrationen aus dem Bereich von 0,001 bis 10 Gew.-%, bezogen auf das Gesamtgewicht der Formulierung, zu wählen.

Es ist dem Fachmann natürlich bekannt, dass kosmetische Emusionen zumeist nicht ohne die üblichen Hilfs- und Zusatzstoffe denkbar sind. Die erfindungsgemäßen kosmetischen Emusionen können dementsprechend - je nach gewünschtem Anwendungszweck - ferner kosmetische Hilfsstoffe enthalten, wie sie üblicherweise in solchen Zubereitungen verwendet werden, beispielsweise Konsistenzgeber, Stabilisatoren, Füllstoffe, Konservierungsmittel, Parfüme, Substanzen zum Verhindern des Schäumens, Farbstoffe, Pigmente, die färbende Wirkung haben, oberflächenaktive Substanzen, Emulgatoren, weichmachende, anfeuchtende und/oder feuchthaltende Substanzen, entzündungshemmende Substanzen, zusätzliche Wirkstoffe wie Vitamine oder Proteine, Lichtschutzmittel, Insektenrepellentien, Bakterizide, Viruzide, Wasser, Salze, antimikrobiell, proteolytisch oder keratolytisch wirksame Substanzen oder andere übliche Bestandteile einer kosmetischen Formulierung wie Alkohole, Polyole, Polymere, Schaumstabilisatoren, organische Lösungsmittel oder auch Elektrolyte.

Letztere können beispielsweise gewählt werden aus der Gruppe der Salze mit folgenden Anionen: Chloride, ferner anorganische Oxo-Element-Anionen. Auch auf organischen Anionen basierende Elektrolyte sind vorteilhaft, z. B. Lactate, Acetate, Benzoate, Citrate, Aminosäuren, Ethylendiamintetraessigsäure und deren Salze und andere mehr. Als Kationen der Salze werden bevorzugt Ammonium,- Alkalimetall-, Erdalkalimetall,- Magnesium-, Eisen- bzw. Zinkionen verwendet. Es bedarf an sich keiner Erwähnung, dass in Kosmetika nur physiologisch unbedenkliche Elektrolyte verwendet werden sollten. Besonders bevorzugt sind Kochsalz, Magnesiumsulfat, Magnesiumchlorid, Zinksulfat und Mischungen daraus.

In einer besonders bevorzugten Ausführungsform enthalten die Emusionen im Sinne der vorliegenden Erfindung ferner einen oder mehrere Wirkstoffe gewählt aus der Gruppe: Ceramid 3, Traubenkernöl, Biotin, Panthenol, Aloe Vera, Hammamelis-Extrakt, Gingko-Extrakt, Honig, Weizenkeimöl, Reiskeimöl, Lotus-Extrakt und Mandelöl.

Die nachfolgenden Beispiele sollen die vorliegende Erfindung verdeutlichen, ohne sie einzuschränken. Alle Mengenangaben, Anteile und Prozentanteile sind, soweit nicht anders angegeben, auf das Gewicht und die Gesamtmenge bzw. auf das Gesamtgewicht der Emusionen bezogen.

### Referenz Beispiele:

| | **W/O-Lotion** | **O/W-Lotion** |
|---|---|---|
| PEG-45/Dodecyl Glycol Copolymer | 0,75 | |
| Methoxy PEG-22/Dodecyl Glycol Copolymer | 0,75 | |
| Ozokerite + PEG-2 Hydrogenated Castor Oil + Sorbitan Isostearate + Hydrogenated Castor Oil | 1 | |
| PEG-7 Hydrogenated Castor Oil | 2 | |
| PEG-40 Stearate | | 1 |
| Glyceryl Stearate | | 2 |
| Phenoxyethanol | 0,5 | |
| Benzyl Alcohol | | 0,5 |
| Propylparaben | 0,1 | 0,1 |
| Ethylparaben | | 0,2 |
| Methylparaben | 0,3 | 0,3 |
| Paraffinum Liquidum | 8 | 3 |
| Glycerin | 3 | 12 |
| Vitis Vinifera | 6 | 6 |
| Octyldodecanol | 5 | |
| Caprylic/Capric Triglyceride | | 3 |
| Oenothera Biennis | 6 | 6 |
| Magnesium Sulfate | 0,5 | |
| Dimethicone | 2 | 2 |
| Silberdihydrogencitrat | 0,3 | 0,15 |
| Aqua | ad 100 | ad 100 |

## Patentansprüche

1. Kosmetische oder dermatologische Emulsionen mit einem Gehalt an
a) Licochalcon A und/oder einem Licochalcon A enthaltenden Extrakt aus *Radix Glycyrrhizae inflatae*
und
b) Silber Dihydrogen Citrat.

2. Emulsion nach Anspruch 1, **dadurch gekennzeichnet, dass** sie - bezogen auf das Gesamtgewicht der Emulsion - 0,0001 bis 5 Gew.-% Licochalcon A enthält.

3. Emulsion nach Anspruch 1, **dadurch gekennzeichnet, dass** sie - bezogen auf das Gesamtgewicht der Emulsion - 0,001 bis 1 Gew.-% Licochalcon A enthält.

4. Emulsion nach Anspruch 1, **dadurch gekennzeichnet, dass** sie - bezogen auf das Gesamtgewicht der Emulsion - 0,005 bis 0,15 Gew.-% Licochalcon A enthält.

5. Emulsion nach einem der vorhergehenden Ansprüche, **dadurch gekennzeichnet, dass** sie einen wässrigen Extrakt aus *Radix Glycyrrhizae inflatae* enthält.

6. Emulsion nach einem der vorhergehenden Ansprüche, **dadurch gekennzeichnet, dass** sie - bezogen auf das Gesamtgewicht der Emulsion - 0,05 bis 0,5 Gew.-% Silber Dihydrogen Citrat enthält.

7. Emulsion nach einem der vorhergehenden Ansprüche, **dadurch gekennzeichnet, dass** sie - bezogen auf das Gesamtgewicht der Emulsion - 0,1 bis 0,3 Gew.-% Silber Dihydrogen Citrat enthält.

8. Emulsion nach einem der vorhergehenden Ansprüche, **dadurch gekennzeichnet, dass** sie ein oder mehrere Derivate ungesättigter Fettsäuren enthält.

9. Emulsion nach Anspruch 8, **dadurch gekennzeichnet, dass** sie Öle enthält, die ungesättigte Fettsäurederivate als Triglyceride enthalten.

10. Emulsion nach einem der vorhergehenden Ansprüche, **dadurch gekennzeichnet, dass** sie in Form einer W/O-Emulsion vorliegt.

11. Emulsion nach einem der vorhergehenden Ansprüche, **dadurch gekennzeichnet, dass** sie W/O-Emulgatoren, gewählt aus der Gruppe PEG-30 Dipolyhydroxystearat, Polyglyceryl-3-Diisostearat, PEG-40 Sorbitan Perisostearate und Cetyl PEG/PPG-10/1 Dimethicone, enthält.

12. Emulsion nach einem der vorhergehenden Ansprüche, **dadurch gekennzeichnet, dass** sie in Form einer O/W-Emulsion vorliegt.

13. Emulsion nach einem der vorhergehenden Ansprüche, **dadurch gekennzeichnet, dass** sie O/W-Emulgatoren, gewählt aus der Gruppe Glycerylstearat, PEG-40 Stearat, Triglycerinmethylglucosedistearat, Polyglyceryl-3 Methylglucose Distearate, Polyethylenglycol-(21)stearylether, Polyethylenglycol(2)stearylether, Cetearylglucosid, Glycerylstearatcitrat, Natriumcetearylsulfat, Cetearylalkohol und/oder Sorbitanstearat enthält.

## Claims

1. Cosmetic or dermatological emulsions with a content of
a) licochalcone A and/or an extract from *Radix Glycyrrhizae inflatae* containing licochalcone A
and
b) silver dihydrogen citrate.

2. Emulsion according to Claim 1, **characterized in that** it comprises - based on the total weight of the emulsion - 0.0001 to 5% by weight of licochalcone A.

3. Emulsion according to Claim 1, **characterized in that** it comprises - based on the total weight of the emulsion - 0.001 to 1% by weight of licochalcone A.

4. Emulsion according to Claim 1, **characterized in that** it comprises - based on the total weight of the emulsion - 0.005 to 0.15% by weight of licochalcone A.

5. Emulsion according to one of the preceding claims, **characterized in that** it comprises an aqueous extract from *Radix Glycyrrhizae inflatae*.

6. Emulsion according to one of the preceding claims, **characterized in that** it comprises - based on the total weight of the emulsion - 0.05 to 0.5% by weight of silver dihydrogen citrate.

7. Emulsion according to one of the preceding claims,
**characterized in that** it comprises - based on the total weight of the emulsion - 0.1 to 0.3% by weight of silver dihydrogen citrate.

8. Emulsion according to one of the preceding claims, **characterized in that** it comprises one or more derivatives of unsaturated fatty acids.

9. Emulsion according to Claim 8, **characterized in that** it comprises oils which contain unsaturated fatty acid derivatives as triglycerides.

10. Emulsion according to one of the preceding claims, **characterized in that** it is in the form of an W/O emulsion.

11. Emulsion according to one of the preceding claims, **characterized in that** it comprises W/O emulsifiers selected from the group consisting of PEG-30 dipolyhydroxystearate, polyglyceryl-3-diisostearate, PEG-40 sorbitan perisostearate and cetyl PEG/PPG-10/1 dimethicone.

12. Emulsion according to one of the preceding claims, **characterized in that** it is in the form of an O/W emulsion.

13. Emulsion according to one of the preceding claims, **characterized in that** it comprises O/W emulsifiers selected from the group consisting of glyceryl stearate, PEG-40 stearate, triglycerol methylglucose distearate, polyglyceryl-3 methylglucose distearate, polyethylene glycol(21) stearyl ether, polyethylene glycol(2) stearyl ether, cetearyl glucoside, glyceryl stearate citrate, sodium cetearyl sulphate, cetearyl alcohol and/or sorbitan stearate.

## Revendications

1. Emulsions cosmétiques ou dermatologiques présentant une teneur en
a. Licochalcone A et/ou un extrait contenant de la Licochalcone A de Radix Glycyrrhizae inflatae
et
b. dihydrogénocitrate d'argent.

2. Emulsion selon la revendication 1, **caractérisée en ce qu'**elle contient - par rapport au poids total de l'émulsion - 0,0001 à 5% en poids de Licochalcone A.

3. Emulsion selon la revendication 1, **caractérisée en ce qu'**elle contient - par rapport au poids total de l'émulsion - 0,001 à 1% en poids de Licochalcone A.

4. Emulsion selon la revendication 1, **caractérisée en ce qu'**elle contient - par rapport au poids total de l'émulsion - 0,005 à 0,15% en poids de Licochalcone A.

5. Emulsion selon l'une quelconque des revendications précédentes, **caractérisée en ce qu'**elle contient un extrait aqueux de Radix Glycyrrhizae inflatae.

6. Emulsion selon l'une quelconque des revendications précédentes, **caractérisée en ce qu'**elle contient - par rapport au poids total de l'émulsion - 0,05 à 0,5% en poids d'hydrogénocitrate d'argent.

7. Emulsion selon l'une quelconque des revendications précédentes, **caractérisée en ce qu'**elle contient - par rapport au poids total de l'émulsion - 0,1 à 0,3% en poids d'hydrogénocitrate d'argent.

8. Emulsion selon l'une quelconque des revendications précédentes, **caractérisée en ce qu'**elle contient un ou plusieurs dérivés d'acides gras insaturés.

9. Emulsion selon la revendication 8, **caractérisée en ce qu'**elle contient des huiles qui contiennent des dérivés d'acides gras insaturés comme triglycérides.

10. Emulsion selon l'une quelconque des revendications précédentes, **caractérisée en ce qu'**elle se trouve sous forme d'une émulsion E/H.

11. Emulsion selon l'une quelconque des revendications précédentes, **caractérisée en ce qu'**elle contient des émulsifiants E/H choisis dans le groupe formé par le dipolyhydroxystéarate de PEG-30, le diisostéarate de polyglycéryle-3, le perisostéarate de PEG-40 et de sorbitane et le cétyl-PEG/PPG-10/1-diméthicone.

12. Emulsion selon l'une quelconque des revendications précédentes, **caractérisée en ce qu'**elle se trouve sous forme d'une émulsion H/E.

13. Emulsion selon l'une quelconque des revendications précédentes, **caractérisée en ce qu'**elle contient des émulsifiants H/E choisis dans le groupe constitué par le stéarate de glycéryle, le stéarate de PEG-40, le distéarate de triglycérolméthylglucose, le distéarate de polyglycéryl-3-méthylglucose, le polyéthylèneglycol-(21)stéaryléther, le polyéthylèneglycol(2)stéaryléther, le cétéarylglucoside, le stéarate-citrate de glycéryle, le cétéarylsulfate de sodium, l'alcool cétéarylique et/ou le stéarate de sorbitane.
